(19) Europäisches Patentamt
European Patent Office
Office européen des brevets

(11) **EP 3 473 317 A1**

(12) **EUROPEAN PATENT APPLICATION**
published in accordance with Art. 153(4) EPC

(43) Date of publication:
**24.04.2019 Bulletin 2019/17**

(21) Application number: **17815366.4**

(22) Date of filing: **19.06.2017**

(51) Int Cl.:
**B01D 12/00** (2006.01)  **B01J 19/00** (2006.01)
**B03C 1/00** (2006.01)  **C12M 1/00** (2006.01)
**G01N 1/28** (2006.01)  **G01N 37/00** (2006.01)

(86) International application number:
**PCT/JP2017/022554**

(87) International publication number:
**WO 2017/221898 (28.12.2017 Gazette 2017/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **20.06.2016 JP 2016121791**

(71) Applicant: **Toppan Printing Co., Ltd.
Tokyo 110-0016 (JP)**

(72) Inventor: **ARAI Noriaki
Tokyo 110-0016 (JP)**

(74) Representative: **Cabinet Plasseraud
66, rue de la Chaussée d'Antin
75440 Paris Cedex 09 (FR)**

(54) **METHOD FOR REPLACING LIQUID MEDIUM AND FLOW PATH DEVICE FOR SAID METHOD**

(57) An objective of the present invention is to replace a liquid medium. A method and a flow channel device for the method are provided. The method includes: a step of forming a laminar flow of a plurality of laminar flow segments by at least two or more liquids, of a sample liquid and a second liquid medium, the sample liquid being a first liquid medium that contains dispersed object particles, and the laminar flow including at least a first laminar flow segment composed of at least the sample liquid, and a second laminar flow segment composed of the second liquid medium; a step of displacing the object particles from the first laminar flow segment to the second laminar flow segment by applying an external force to the laminar flow, the object particles having at least one physical characteristic of being allowed to displaced towards the second laminar flow segment from the first laminar flow segment by an external force; and a step of recovering a laminar flow fraction containing the object particles from the second laminar flow segment, the laminar flow fraction being recovered from a laminar flow fraction recovery surface perpendicular to the flow direction of the laminar flow, and the laminar flow fraction recovery surface being separated from the cross section of the first laminar flow segment.

FIG.1

1/14

## Description

[Technical Field]

The present invention relates to a method of replacing a liquid medium, and in particular to a method of replacing a liquid medium of a mixed fluid sample containing at least one of bioparticles, beads, and composite particles of the bioparticles and beads, as object particles.

[Background Art]

Separating object particles from a mixed fluid sample (e.g., blood, culture solution, reaction solution) for replacement of the liquid medium is a technique required for basic research, diagnosis, and treatment.

For example, a liquid medium of proteins or the like contained in a mixed fluid sample is typically replaced by a method of capturing the proteins or the like by antibody-labeled magnetic particles to form composite particles, followed by capturing the composite particles by magnetic force, and then repeating removal and cleaning the supernatant to replace the liquid medium with a desired liquid medium.

Other methods known to be usable for replacement of a liquid medium include a flow cytometric method, and a method of separating cells in which cells (e.g., leukocytes and erythrocytes) having intrinsic density (specific gravity) are separated by centrifugation or the like.

For a mixed fluid sample containing object particles, a typical conventional method used for accurately replacing the liquid medium is to iterate separation of solids from the liquid by centrifugation or the like, removal of the separated liquid, and addition of a new liquid medium.

Such a conventional method has an issue that the object particles are partially lost in the process thereof. In order to achieve accurate replacement of a liquid medium, it is required to repeatedly conduct cleaning. However, repetition of cleaning may result in reduction of the recovery ratio of the object particles when the mixed sample contains fewer object particles.

[Citation List]

[Non-Patent Literature]

NPL1: D.W.Inglis, et al, Critical particle size for fractionation by deterministic lateral displacement, Lab on a Chip, 6, 655-658 (2006).

[Summary of the Invention]

[Technical Problem]

Therefore, an objective of the present invention is to provide a method of accurately replacing a liquid medium with a desired liquid medium while minimizing the loss of object particles contained in a liquid sample, and to provide a device for the method.

[Solution to Problem]

[First aspect of the present invention]

A first aspect of the present invention is a method of replacing a liquid medium by displacing object particles from a first liquid medium into a second liquid medium, including:

> (i) a step of forming a laminar flow of a plurality of laminar flow segments by feeding at least two or more liquids, of a sample liquid and a second liquid medium, in the same direction,
> the sample liquid being a first liquid medium that contains dispersed object particles,
> the laminar flow including at least a first laminar flow segment composed of the sample liquid, and a second laminar flow segment composed of the second liquid medium;
> (ii) a step of displacing the object particles from the first laminar flow segment to the second laminar flow segment by applying an external force to the laminar flow, the object particles having at least one physical characteristic of being allowed to displace towards the second laminar flow segment from the first laminar flow segment by an external force; and
> (iii) a step of recovering a laminar flow fraction containing the object particles from the second laminar flow segment, the laminar flow fraction being recovered from a laminar flow fraction recovery surface perpendicular to a flow direction of the laminar flow, and the laminar flow fraction recovery surface being separated from the cross section of the first laminar flow segment.

[Second aspect of the present invention]

A second aspect of the present invention is a flow channel device that displaces object particles from a first liquid medium to a second liquid medium, including:

> (i) a plurality of inlets including at least a first inlet for introducing a sample liquid, and a second inlet for introducing a second liquid medium on the upstream side of the flow channel device;
> (ii) a flow channel that forms a laminar flow;
> (iii) two or more outlets including at least a first outlet and a second outlet on the downstream side of the flow channel device; and
> (iv) a mechanism that applies an external force.

In the flow channel device: the sample liquid is a first liquid medium containing dispersed object particles;

the flow channel forms a laminar flow including at least a first laminar flow segment composed of the sample liquid, and a second laminar flow segment composed of a second liquid medium;

the second outlet is an object particle recovery outlet that recovers a laminar flow fraction from a laminar flow fraction recovery surface perpendicular to the flow direction of the laminar flow, the laminar flow fraction including the object particles that has been displaced to the second laminar flow segment by the external force;

the first outlet is on the first laminar flow segment side as viewed from the second outlet; and

a partition wall of the second outlet on the first laminar flow segment side is provided such that the laminar flow fraction recovery surface is separated from the cross section of the first laminar flow segment.

[Advantageous Effects of the Invention]

According to the present invention, a liquid medium can be accurately replaced while the loss of object particles is further minimized compared with the prior art.

Further, in the present invention, the liquid medium can be continuously replaced.

[Brief Description of the Drawings]

Fig. 1 is a schematic diagram illustrating an example of a method of replacing a liquid medium, according to an embodiment of the present invention.

Fig. 2 is a schematic diagram illustrating an example of the method of replacing a liquid medium by using three or more laminar flow segments, according to an embodiment of the present invention.

Fig. 3 is a schematic diagram illustrating an example of a basic structure of a flow channel device according to an embodiment of the present invention.

Fig. 4 is a schematic diagram illustrating an example of the flow channel device according to an embodiment of the present invention.

Fig. 5 is a schematic diagram illustrating an example of a basic structure of a flow channel device with three or more laminar flow segments according to an embodiment of the present invention.

Fig. 6 is a schematic vertical cross-sectional view illustrating an example of the flow channel device according to an embodiment of the present invention.

Fig. 7 is a schematic diagram illustrating an example of a flow channel device including a liquid feeding unit and a recovering unit according to an embodiment of the present invention.

Fig. 8 is a schematic diagram illustrating an example of a basic principle of an external force for displacing particles according to an embodiment of the present invention.

Fig. 9 is a schematic diagram illustrating an example of the flow channel device including a deterministic lateral displacement (DLD) structure according to an embodiment of the present invention.

Fig. 10 is a schematic vertical cross-sectional view illustrating an example of the flow channel device including a deterministic lateral displacement (DLD) structure, according to an embodiment of the present invention.

Fig. 11 is a schematic diagram illustrating an example of the flow channel device of an embodiment of the present invention.

Fig. 12 is a photograph showing an example of a flow channel device used in an embodiment of the present invention.

Fig. 13 is a photograph showing a detailed structure of an example of the flow channel device used in an embodiment of the present invention.

Fig. 14 is a photograph showing an example of a process of formation of laminar flow in the flow channel device used in an embodiment of the present invention.

[Description of the Embodiments]

[1. Liquid medium replacement method of the first aspect of the present invention]

With reference to the drawings, a liquid medium replacement method of the present invention will be described.

The present invention provides a method of replacing a liquid medium (first liquid medium) of a mixed fluid sample (sample liquid) with a desired second liquid medium while minimizing loss of the object particles, the mixed fluid sample containing object particles 1 (bioparticles including, for example, cells and the like, beads, composite particles of beads and bioparticles).

The sample liquid refers to a liquid medium (first liquid medium) containing dispersed object particles, and may be a mixed fluid sample containing the object particles, or may be a liquid obtained by appropriately diluting the mixed fluid sample with a desired buffer solution. For example, the sample liquid may be a body fluid such as blood containing blood cells, lymph, saliva, urine, tears, or the like.

The term first liquid medium as used herein refers to all the rest of the sample liquid as a result of removing the object particles therefrom. The first liquid medium herein refers to not only a pure liquid medium, but also one that contains components other than the object particles.

Examples of the object particles suspended in a liquid medium (culture solution or the like) include bioparticles, beads, and composite particles of beads and bioparticles. More specific examples of the bioparticles include biomolecules (e.g., nucleic acids, proteins, sugars, lipids, etc.), extracellular vesicles (e.g., exosomes, apoptotic bodies, etc.), and cells. Thus, the object particles may be those of a single substance or may be those of a composite of two or more substances. The type and the number of object particles contained in the sample liquid are not limited. Examples of the beads that form the composite particles include beads to which target capturing molecules that recognize the characteristic structure of bioparticles are bound. As such target capturing molecules, antibodies, peptide aptamers, lectins, intercellular adhesion molecules, sugar chains, or cell-recognizing macromolecules are preferable. Further, as beads carrying such target capturing molecules, polystyrene beads, latex beads, magnetic beads, or the like are preferable. It should be noted that the present invention is not limited to these examples, and may use any means which is appropriately selected to achieve the present object.

The second liquid medium may be any liquid medium suitable for receiving the object particles. However, if the object particles contain cells and an effect on the viability of the cells is to be avoided, isotonic solutions may be used singly or in combination of two or more. For example, physiological saline solution, phosphate buffer solution (PBS) or the like may be used. When the object particles contain nucleic acids, proteins, or the like, a buffer solution suitable for avoiding denaturation of these substances is preferably selected. Further, the second liquid medium may be one that dissociates the composite particles to liberate the object particles by adjusting the pH or the like. To dissolve the object particles in the liquid medium, or to arbitrarily cause reaction of the object particles with the liquid medium, a suitable liquid medium may be appropriately selected as necessary, and is not limited to the liquid medium set forth above.

The liquid medium replacement method of the present invention uses a laminar flow including at least a first laminar flow segment composed of a sample liquid, and a second laminar flow segment composed of the second liquid medium, the first laminar flow segment and the second laminar flow segment flowing in the same direction in parallel to each other, and replaces the first liquid medium containing the object particles with the second liquid medium by displacing the object particles towards the second laminar flow segment from the first laminar flow segment by an external force.

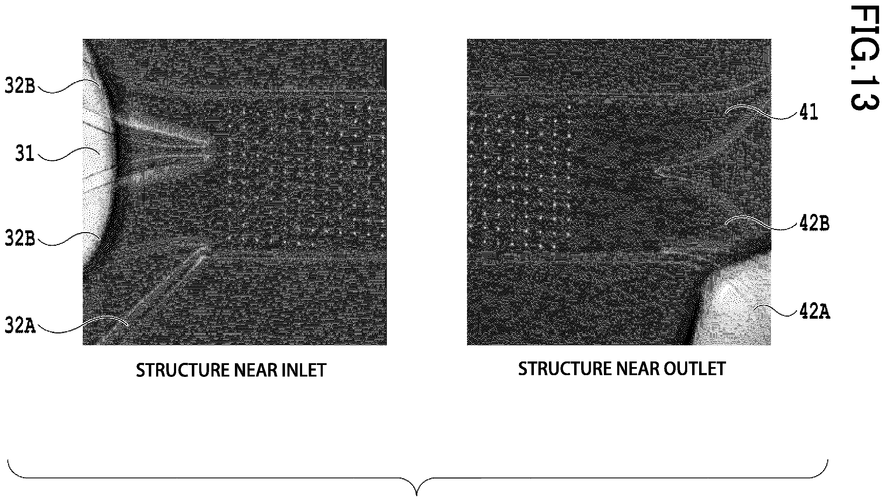

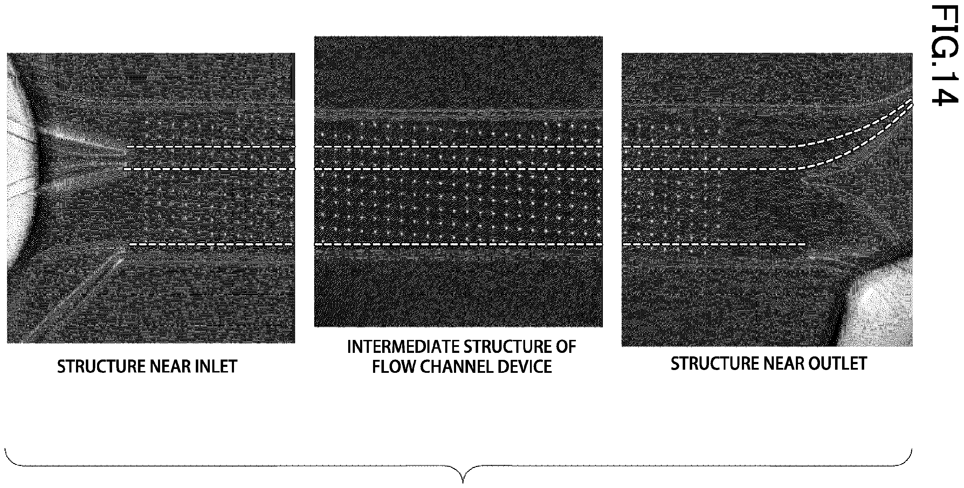

The term laminar flow used herein refers to a smooth flow in which, when a viscous fluid is passed through a tube, the fluid portions move parallel to the tube axis in an orderly manner, without causing mixing between adjacent fluid portions. In contrast, a flow which is not a laminar flow is referred as a turbulent flow in which adjacent fluid portions are mixed with each other. Using such a property of the laminar flow in which the adjacent fluid portions do not mix with other, the liquid medium can be accurately replaced. The laminar flow used in the method of the present invention includes at least a first laminar flow segment composed of at least a sample liquid, and a second laminar flow segment composed of a second liquid medium which is a desired liquid medium. The term laminar flow segment used herein refers to a laminar flow composed of a predetermined liquid medium, and flowing as an integral body at a predetermined flow rate. Therefore, when a laminar flow includes liquid medias different from each other (e.g., phosphate buffer solutions whose contents other than the object particles are different from each other), or when a laminar flow includes flows whose rates are different from each other (if liquid media are fed by respective different feeding devices, their flow rates are considered to be different from each other), laminar flows flowing parallel to each other are taken to be laminar flow segments separate from each other. When a laminar flow includes only one liquid medium and the liquid medium is fed by the same liquid feeding device, but two spatially separated flows are formed because the liquid medium inlet connected to the liquid feeding device is branched into two, these flows are taken to be separate laminar flow segments (see the inlet 32B in Figs. 13 and 14). However, in terms of not disturbing the laminar flow as much as possible, it is preferable that the mutually different laminar flow segments substantially have the same flow rate per unit cross-sectional area.

In the method of the present invention, the object particles have at least one physical characteristic of being displaced towards the second laminar flow segment by an external force. Preferred external forces may be at least one of external forces due to electric field, magnetic field, hydrodynamic filtration, or deterministic lateral displacement (DLD), with the corresponding physical characteristics of the object particles being electric charge, magnetism, and particle size, respectively. For example, when the object particles are magnetic particles, the object particles can be gradually displaced from the first laminar flow segment composed of the sample liquid to the second laminar flow segment composed of the desired liquid medium (second liquid medium) by applying a magnetic field in a direction perpendicular to the flow direction on the laminar flow plane. As a hydrodynamic method, for example, a structure based on the principle of DLD may be provided in the flow channel device, so that object particles can similarly be displaced to the second laminar flow segment composed of the object liquid medium (second liquid medium) according to the size of the object particles.

For example, the physical characteristics of such object particles may utilize the physical properties of the bioparticles themselves, but may be one that is derived from the physical properties of the beads when the object particles are the composite particles of the beads and the bioparticles. For example, by forming composite particles of beads and object bioparticles, at least the particle size can be adjusted.

More specifically, the liquid medium replacement method of the present invention includes: (i) a step of forming a laminar flow including at least a first laminar flow segment composed of a sample liquid, and a second laminar flow segment composed of a second liquid medium; (ii) a step of displacing the object particles from the sample liquid to the second liquid medium by applying an external force; and (iii) a step of recovering the laminar flow fractions of the displaced object particles.

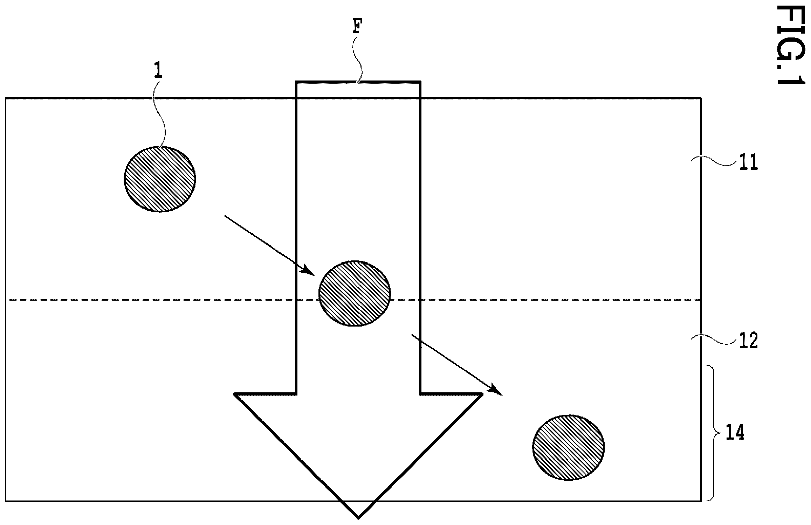

Fig. 1 shows an example of a laminar flow in which a first laminar flow segment 11 composed of a sample liquid is adjacent to a second laminar flow segment 12 composed of a second liquid medium which is a desired liquid medium. Further, Fig. 1 shows a process of displacing the object particles 1 from the first laminar flow segment 11 to the second laminar flow segment 12 by an external force F. In Fig. 1, a reference sign 14 indicates a laminar flow fraction recovery surface which is a plane perpendicular to the flow direction of the laminar flow, and the object particles are recovered by recovering the laminar flow fractions of the second laminar flow segment 12 passing through the laminar flow fraction recovery surface.

In the method of the present invention, the laminar flow fraction recovery surface is separated from the cross section of the first laminar flow segment. More preferably, the laminar flow fraction recovery surface is provided within the cross section of the second laminar flow segment to avoid intrusion of the sample liquid as much as possible when the laminar flow fraction is recovered. Herein, the cross section of the first laminar flow segment refers to a cross section perpendicular to the laminar flow direction of the first laminar flow segment. The degree of separation between the laminar flow fraction recovery surface and the cross section of the first laminar flow segment may be determined in consideration of the turbulence of the laminar flow on the laminar flow fraction recovery surface, the influence of the movement of the liquid medium due to the diffusion at the laminar flow interface, and the like. In an example shown in Fig. 1, the laminar flow fraction recovery surface is provided at least within a cross section perpendicular to the laminar flow direction of the second laminar flow segment.

In terms of obtaining a sufficient recovery rate of the object particles, it is preferable that a laminar flow fraction recovery surface is ensured to be sufficiently large to recover the object particles as completely as possible. The laminar flow fraction recovery surface is preferably designed to have a sufficient area for recovering 90% or more of the object particles, more preferably 95% or more, and most preferably 99% or more. Therefore, it is advantageous to design the laminar flow fraction recovery surface so that distribution of the object particles would be as narrow as possible, by adjusting the flow rate of the laminar flow, the length of the flow channel, the displacement speed of the object particles caused by the external force, and the like. In particular, it is advantageous to design a sufficiently long flow channel.

The configuration of the laminar flow is not limited to one with two laminar flow segments as shown in Fig. 1, and may be one with three or more laminar flow segments as necessary.

For example, by providing a third laminar flow segment between the first and second laminar flow segments, the influence of the movement of the liquid medium components due to diffusion at the laminar flow interface can be further reduced. In this case, the first laminar flow segment is composed of the sample liquid, the second laminar flow segment is composed of the desired liquid medium (second liquid medium), and the third laminar flow segment is composed of any other liquid medium (third liquid medium). With this structure, contamination of the components can be reduced, and the contaminants, besides the object particles, can be cleaned during the process of passing particles through the third laminar flow segment. Therefore, when the liquid medium is desired to be replaced with higher accuracy, it is further preferable that the laminar flow is configured to include the third laminar flow segment interposed between the first and second laminar flow segments.

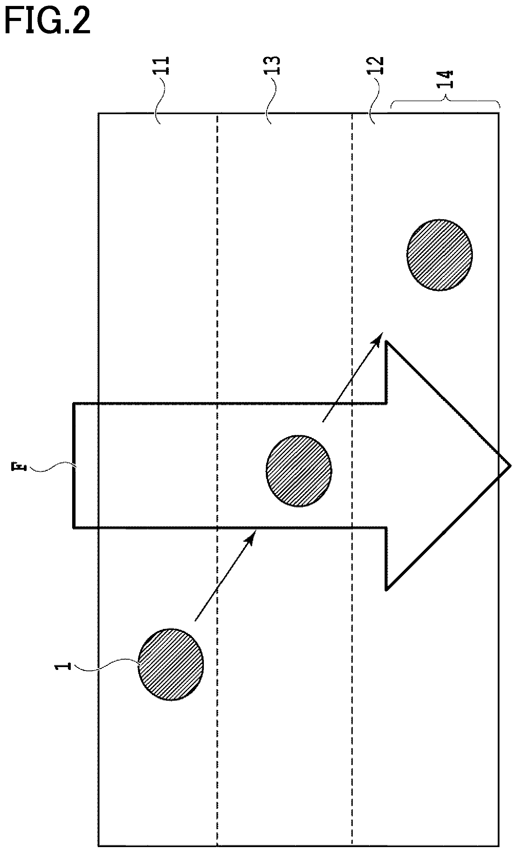

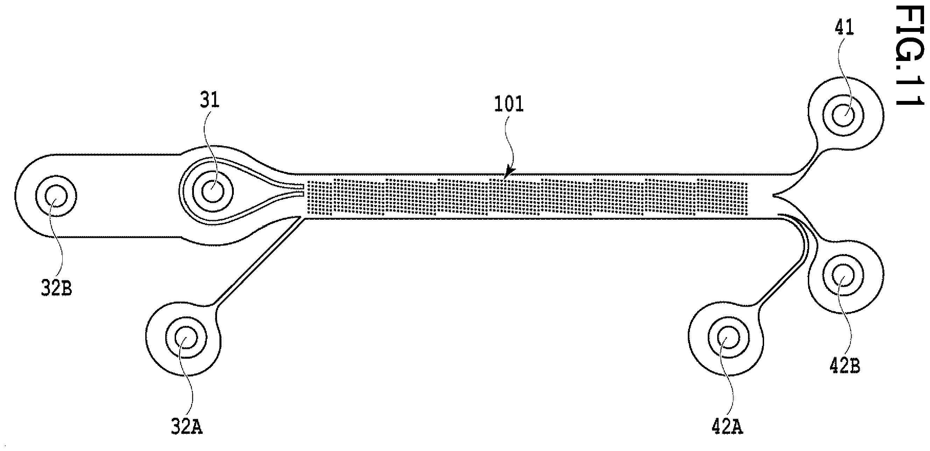

An example of such a configuration is shown in Fig. 2. That is, Fig. 2 illustrates an example in which, in addition to the first and second laminar flow segments 11 and 12, the laminar flow further includes a third laminar flow segment 13 interposed therebetween. In this configuration, the first laminar flow segment 11 is composed of the sample liquid, and the second laminar flow segment 12 is composed of the second liquid medium which is the liquid medium to be replaced, and the third laminar flow segment 13 is composed of the third liquid medium. Figs. 11, 13 and 14 are also included in the example of this configuration.

Fig. 2 shows an example in which a laminar flow fraction recovery surface 14 is provided within the cross section of the second laminar flow segment similarly to Fig. 1. However, in the example of the configuration shown in Fig. 2, the laminar flow fraction 14 does not necessarily have to be within the cross section perpendicular to the laminar flow direction of the second laminar flow segment, for separation from the cross section of the first laminar flow segment, but may include part of the cross section perpendicular to the laminar flow direction of the third laminar flow segment 13. In particular, when the third and second liquid mediums are the same liquid mediums, the laminar flow fraction recovery surface 14 may still include part of the cross section perpendicular to the laminar flow direction of the third laminar flow segment 13. However, in terms of replacing the liquid medium with high accuracy as described above, the laminar flow fraction recovery surface is preferably provided so as to be at least flush with the cross section perpendicular to the laminar flow direction of the second laminar flow segment, or more preferably is provided within the cross section.

The configuration of the laminar flow used in the present invention is not limited to the examples shown in Figs. 1 and 2, and the laminar flow may be configured to include the additional laminar flow segment at any other arbitrary position. For example, the laminar flow may be configured to include the third laminar flow segment at a position adjacent to the second laminar flow segment on the other side thereof opposite to the side facing the first laminar flow segment, the third laminar flow segment being composed of the third liquid medium. Such an example of the configuration of the laminar flow may be advantageously used. For example, let us assume that bioparticles used as the object particles are a composite of bioparticles and beads, a liquid medium used as the second liquid medium is one that dissociates the composite particles with the adjustment of pH or the like for liberation of the object bioparticles, and the external force depends on the properties of the beads (e.g., magnetism of the beads). In this case, the beads liberated in the second laminar flow segment are further displaced to the third laminar flow segment by the external force. However, since the external force no longer acts on the object bioparticles themselves, the object bioparticles remain in the second laminar flow segment. Therefore, only the object bioparticles can be directly recovered from the second laminar flow segment. Further, in this case, it is preferable that the laminar flow fraction recovery surface is not only separated from the cross section of the first laminar flow segment, but also separated from the cross section of the third laminar flow segment within a limitation necessary for avoiding the intrusion of beads. Further, the laminar flow fraction recovery surface used in this case is preferably provided within the cross section of the second laminar flow segment.

[2. Flow channel device of the second aspect of the present invention]

The flow channel device suitably used in the method of replacing the liquid medium according to the first aspect of the present invention described in the above item 1 includes:

> (i) a plurality of inlets including at least a first inlet for introducing a sample liquid, and a second inlet for introducing a second liquid medium, on the upstream side of the flow channel device;
> (ii) a flow channel that forms a laminar flow;
> (iii) two or more outlets including at least a first outlet and a second outlet on the downstream side of the flow channel device; and
> (iv) a mechanism that applies an external force, wherein,

the flow channel forms a laminar flow that includes at least a first laminar flow segment composed of the sample liquid, and a second laminar flow segment composed of the second liquid medium,
the second outlet serves as an object particle recovery outlet that recovers laminar flow fractions from a laminar flow fraction recovery surface perpendicular to the flow direction of the laminar flow, the laminar flow fractions including object particles that have been displaced to the second laminar flow segment by an external force,

the first outlet is on the first laminar flow segment side as viewed from the second outlet, and
a partition wall of the second outlet on the first laminar flow segment side is provided such that the laminar flow fraction recovery surface is separated from the cross section of the first laminar flow segment.
Herein, when the first outlet is adjacent to the second outlet, the partition wall of the second outlet on the first laminar flow segment side separates the first outlet from the second outlet. However, when an additional outlet (e.g., third outlet) is interposed between the first outlet and the second outlet, and the additional outlet is adjacent to the second outlet, the partition wall of the second outlet on the first laminar flow segment side separates the additional outlet from the second outlet.
Flow channels usable for the flow channel device are not particularly limited as long as they have a mechanism of generating a laminar flow. However, depending on the width or flow rate of the flow channel, a turbulence (mixed flow) might be generated instead of a laminar flow. In that case, the flow channel should be used under the conditions for forming laminar flow.
It should be noted that whether the flow occur as a laminar flow or a turbulent flow is known to depend on a dimensionless number that is an index obtained by dividing an inertial force referred to as a Reynolds number by a viscous force. For example, in the case of flow in a circular tube, the critical Reynolds number, which is a boundary between laminar flow and turbulent flow, is said to be about 2000 to 4000, according to general criteria. When the fluid has a large viscosity coefficient, or has a small flow rate, the viscosity force becomes dominant and tends to cause the fluid to be a laminar flow. Further, in a micro (channel) device having a microstructure using a micro processing technique of an integrated circuit, the Reynolds number is generally considered to be small, and therefore a laminar flow is obtained.
Also, recovering the laminar flow fractions from the laminar flow fraction recovery surface means recovering the laminar flow segment passing through the laminar flow fraction recovery surface.
With reference to the drawings, the flow channel device of the present invention will be described.

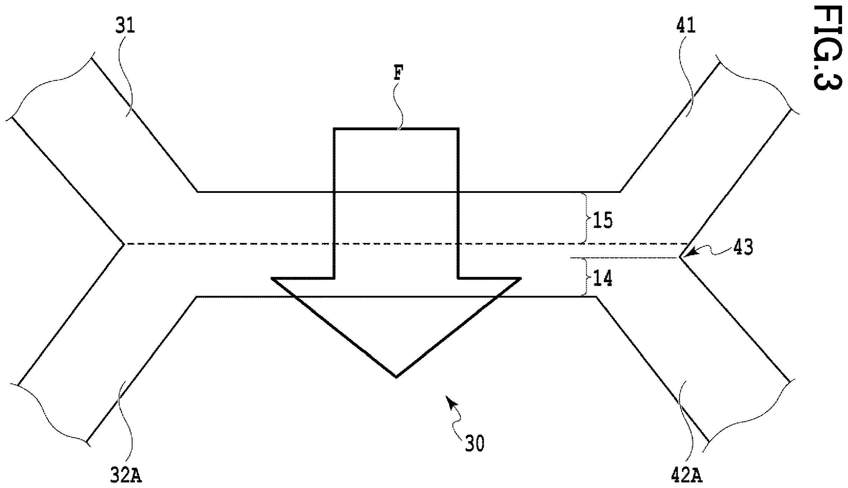

Fig. 3 is a schematic view of an example of a basic structure 30 of the flow channel device of the present invention as viewed from above. This flow channel device forms a laminar flow constituted by a first laminar flow segment composed of a sample liquid, and a second laminar flow segment composed of a second liquid medium that is a desired liquid medium.
First, the inlet of the flow channel device of Fig. 3 is configured to include a sample liquid inlet 31 and a liquid medium (second liquid medium) inlet 32A. The fluid outlet of the flow channel device of Fig. 3 is configured to include a first outlet 41 mainly for discharging the sample liquid, and a second outlet 42A for discharging the desired liquid medium (second liquid medium). The number of the inlets and the outlets is not necessarily limited to two as shown in Fig. 1, and more inlets and outlets may be provided depending on the purpose.

As also shown in Fig. 3, a mechanism F that provides an external force for displacing the object particles is provided between the sample liquid inlet 31 and the liquid medium inlet 32A, and the first outlet 41 and the second outlet 42A of the channel device. With this mechanism, the object particles contained in the sample liquid can be displaced in a direction orthogonal to the flow direction on the laminar flow plane.

Several mechanisms F, which applies an external force, of the same design may be regularly provided in succession along the flow channel. Alternatively, several mechanisms may be provided along the fluid channel with the design gradually changed by appropriately combining methods according to the purpose. As described above, the external force is preferably at least one of external forces due to electric field, magnetic field, hydrodynamic filtration, and deterministic lateral displacement (DLD). However, the external force is not particularly limited thereto as long as it can displace the object particles.

Fig. 3 also shows the laminar flow fraction recovery surface 14 provided on a plane perpendicular to the laminar flow. A partition wall 43 is provided such that the laminar flow fraction recovery surface 14 is separated from the cross section 15 of the first laminar flow segment to prevent intrusion of the liquid media other than the desired liquid media. In the example shown in Fig. 3, the laminar flow fraction recovery surface 14 is provided within the cross section of the second laminar flow segment.

However, the laminar flow fraction recovery surface 14 preferably has an area sufficient for recovering nearly all the object particles. Therefore, it is advantageous to design the laminar flow fraction recovery surface to cause distribution of the object particles as narrow as possible by adjusting the flow rate of the laminar flow, the length of the flow channel, the displacement speed of the object particles due to the external force, and the like. In particular, it is advantageous to design the length of the flow channel to be sufficiently long.

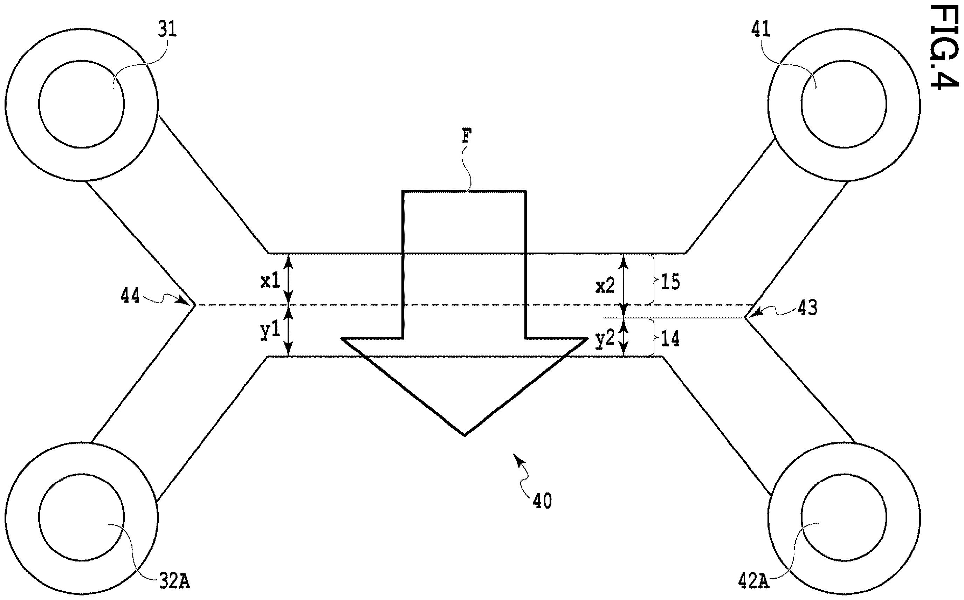

Fig. 4 illustrates an example of a flow channel device in which a partition wall is provided so that two inlets and two outlets of the flow channel device of Fig. 3 are independent from each other. Since it is necessary to supply the sample liquid as a laminar flow with a desired liquid medium (second liquid medium), the entrance part of the sample liquid inlet 31 through which a liquid medium flows into a flow channel device 40 is preferably configured to have independent partition walls as shown in the drawing. The sample liquid fed from the sample liquid inlet 31 flows while maintaining a laminar flow (flow forming a layer parallel to the flow direction) with the second liquid medium (typically a buffer solution) fed from the liquid medium inlet 32A, and the object particles are displaced in a direction oblique to the flow direction according to the mechanism that applies the external force described above. Accordingly, the object particles are displaced from the laminar flow segment of the sample liquid to the laminar flow segment of the second liquid medium, which is the desired liquid medium. On the other hand, particles other than the object particles flow in a generally straight line following the flow of the laminar flow segment (first laminar flow segment) of the sample liquid.

Thus, for the purpose of more effectively replacing the liquid medium, the sample liquid inlet 31 in the direction perpendicular to the laminar flow on the flow channel device plane is preferably provided not on the second outlet 42A side in the perpendicular direction, but on the first outlet 41 side in the perpendicular direction.

Similarly, the liquid medium (second liquid medium) inlet 32A in the direction perpendicular to the laminar flow on the plane of the flow channel device is preferably provided not on the first outlet 41 side in the perpendicular direction, but on the second outlet 42A side in the perpendicular direction.

Further, the structures of the first outlet 41 and the second outlet 42A may be appropriately changed in design according to the purpose in order to adjust the amount of liquid to be discharged.

For example, when the partition walls 43 in the vicinity of the outlets are provided at a 1:1 interval from the upper part to the lower part in the drawing (when the flow rates of the first and second laminar flow segments are the same, x2:y2 =1:1, Fig. 4), the amounts of liquid obtained from the outlets is approximately 1:1. On the other hand, when the partition wall 43 is similarly provided at a 9:1 interval, the amounts of liquid obtained from the second outlet 42A can be concentrated by approximately 10 times (when the flow rates of the first and second laminar flow segments are the same, x2:y2 =9:1, Fig. 4). Thus, depending on desired use, the flow channel device can be used not only for the replacement of the liquid medium of the object particles, but also for the concentration of the liquid amount.

Similarly, by appropriately changing the inlet (e.g., adjusting the position of the partition wall 44 of Fig. 4), the ratio of the amounts of liquid to be used (when the flow rates of the first and second laminar flow segments are the same, x1: y1) can be changed, which is effective for reducing the amount of liquid to be used.

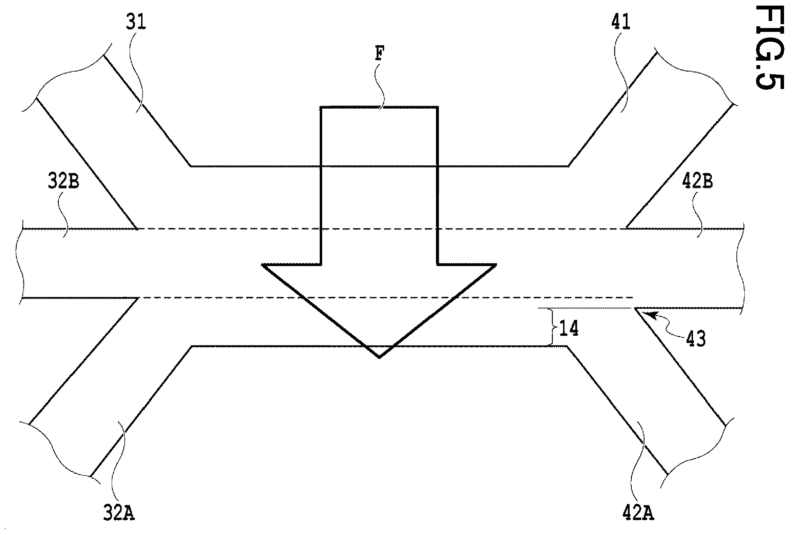

Further, depending on the desired use, three or more laminar flow segments may be configured. In that case, as shown in Fig. 5, the laminar flow may be configured to include a laminar flow segment (third laminar flow segment) of another liquid medium (third liquid medium) between the laminar flow segment of the sample liquid (first laminar flow segment) and the laminar flow segment of the object liquid medium (second liquid medium) (second laminar flow segment).

In this case, the liquid medium (third liquid medium) inlet 32B and a third outlet 42B may be appropriately provided as an inlet and outlet, respectively, for introducing and discharging the third liquid medium. It is suitable for the purpose of the present method to place the liquid medium inlet 32B between the sample liquid inlet 31 and the liquid medium (second liquid medium) inlet 32A. However, the third outlet 42B does not have to be necessarily provided because the first outlet 41 can also serve as the third outlet 42B. However, in terms of achieving the objective of the present invention, it is preferred not to share the second outlet 42A with other liquid media, as described above, to minimize the intrusion of other liquid media into the object liquid medium (second liquid medium).

Similarly, when 4, 5, ..., n or more of the laminar flow segments are provided as required, each laminar flow segment may appropriately have an inlet for introducing the liquid medium and an outlet for discharging the liquid medium. Also, it is preferable, but not always necessary, that these inlets for introducing the liquid mediums (additional liquid mediums other than the second liquid medium) are also provided between the sample liquid inlet 31 and the liquid medium (second liquid medium) inlet 32A. On the other hand, the outlets for the additional liquid mediums do not have to be necessarily provided since they may be replaced by an outlet (e.g., first outlet) other than the second outlets for the second liquid medium.

Fig. 5 shows an example in which the outlet side partition wall 43 is provided such that the laminar flow fraction recovery surface 14 is located within the cross section of the second laminar flow segment similarly to Figs. 3 and 4. However, this is only an example of a preferred embodiment. In the example of Fig. 5, the third laminar flow segment is interposed between the second laminar flow segment and the first laminar flow segment composed of the sample liquid. Therefore, as long as the laminar flow fraction recovery surface 14 is separated from the cross section of the first laminar flow segment, the outlet side partition wall 43 may be provided being offset towards the third laminar flow segment side. However, in terms of more accurately replacing the liquid medium, the laminar flow fraction recovery surface is preferably separated from the cross section of the first laminar flow segment as much as possible, and even more preferably, within the cross section of the second laminar flow segment.

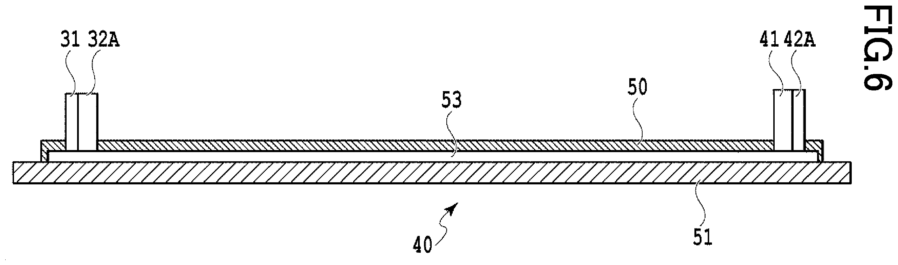

Fig. 6 is a schematic vertical cross-sectional view illustrating an example of a flow channel device according to an embodiment of the present invention.

The flow channel device 40 may be produced by bonding a flow channel structure 50 and a planar structure 51 to each other, with a flow channel space 53 provided therebetween. The flow channel structure 50 includes inlets, outlets, and the like. Further, the configuration of the flow channel device 40 may be appropriately changed by respectively connecting tubes to the sample liquid inlet 31, the liquid medium (second liquid medium) inlet 32A, the first outlet 41, and the second outlet 42A, or by providing joints for connection with a syringe and the like.

The height of the flow channel space 53 is not limited as long as it is a height that allows the object particles to pass therethrough.

The material of the flow channel structure 50 and the method of producing the flow channel structure may be appropriately selected from known ones. Examples of the material of the member may include glass, silicone, dimethylpolysiloxane, plastic, and the like.

Materials used for the planar structure 51, however, are not particularly limited as long as they are flat and can be joined to the flow channel structure 50; but glass, plastic, or the like, if it is strong, is preferably used.

The flow channel device 40 may only have to be provided with a flow channel structure including an inlet, an outlet, and the like, and a flow channel space. For example, the flow channel device 40 may be configured to have a flow channel structure, with an inlet, an outlet, and the like being provided to the planar structure 51 side thereof shown in the drawing.

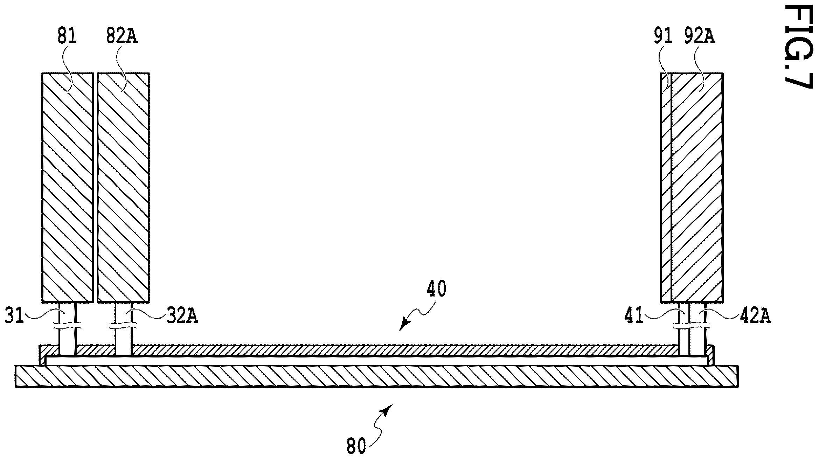

Fig. 7 is a schematic diagram illustrating an example of a flow channel device including a liquid feeding unit and a recovering unit according to an embodiment of the present invention.

A flow channel apparatus 80 is obtained by providing the flow channel device 40 with a plurality of liquid feeding units and recovering units. Fig. 7 illustrates an example in which the sample liquid inlet 31 is provided with a sample liquid feeding unit 81, the liquid medium inlet 32A is provided with a liquid medium feeding unit 82A, the first outlet 41 is provided with a first recovering unit 91, and the second outlet 42A is provided with a second recovering unit 92A.

The sample liquid feeding unit 81 and the liquid medium feeding unit 82A may each have a mechanism including a liquid feeding system, so that liquid can be independently fed at a constant speed. These mechanisms, however, are not particularly limited as long as they are capable of feeding liquid at a constant speed. For example, a mechanism of feeding liquid by a syringe pump or the like is suitable. If necessary, the sample liquid feeding unit 81 may be provided with a stirring mechanism for preventing precipitation and aggregation of particles.

The mechanisms of the first and second recovering units 91 and 92A are not particularly limited as long as they recover the discharged liquid, but a mechanism that can fractionate the discharged liquid immediately before and after the start of use of the device may be provided.

Similarly to the above configuration, when 4, 5,..., n or more of the laminar flow segments are provided as necessary, an inlet and an outlet may be appropriately provided to each liquid medium, and a liquid feeding unit and a recovering unit respectively corresponding to the inlet and the outlet may be appropriately provided.

Furthermore, a plurality of flow channel devices of the present invention may be connected in series. For example, the second outlet of a first flow channel device may be connected to the first inlet of a second flow channel device to be mated, thereby forming a connection. The mechanism or the strength of an external force applied to the plurality of flow channel devices to be used may be different between devices. With this configuration, the object particles can be more accurately displaced.

For example, in separation of bioparticles as object particles, let us assume that particles used as the object particles are a composite of bioparticles and beads, a liquid medium used as the second liquid medium is one that dissociates the composite particles with the adjustment of pH or the like for liberation of the object bioparticles, and the external force depends on the properties of the beads (e.g., magnetism and size of the beads). In this case, by further introducing the bioparticles and the beads, which have been liberated in the second laminar flow segment, into a second flow channel device connected, only the beads can be displaced by the external force of the second flow channel device to directly recover only the object bioparticles. In this case, in the second flow channel device, the bioparticles that do not form the composite are recovered from the first laminar flow segment. The laminar flow fraction recovery surface in the second flow channel device in this case is preferably separated from the cross section of the second laminar flow segment, and more preferably is located within the cross section of the first laminar flow segment.

[3. External forces for displacing particles used in embodiments of the present invention]

Next, the principle of deterministic lateral displacement (DLD) will be described. The deterministic lateral displacement (DLD) is based on an external force for displacing the object particles that have been specifically used in the embodiments of the present invention.

[3-1. Deterministic Lateral Displacement (DLD)]

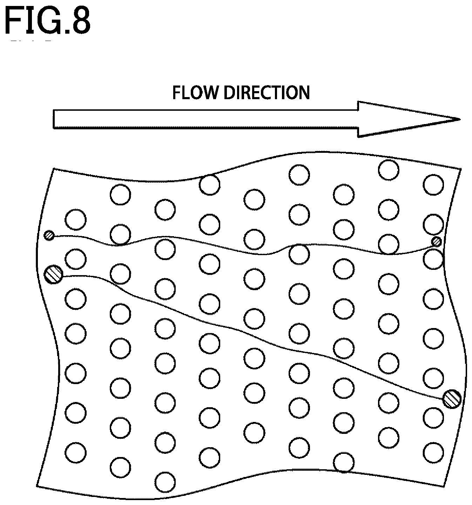

Deterministic lateral displacement (DLD) is a method for sorting particles by particle size thereof by utilizing the properties of the particles that, when a dispersion of the particles passes through columnar structures (pillars) which are slightly offset from each other, large particles flow obliquely due to a change in flow generated around the pillars, while small particles flow substantially linearly with the laminar flow (see Fig. 8 of NPL 1).

When flow channels including obstacles (micropillars) that are arrayed with being offset from each other are used, small particles flow straight with the flow, while large particles flow obliquely in conformity with the offset obstacles with the flow changing around the obstacle. Therefore, by applying the present method to the object particles, an external force that displaces the object particles between the laminar flow segments can be applied to the object particles.

[3-2. Threshold for size of obliquely displaced particles based on DLD principle]

Based on the DLD principle described in NPL 1, the threshold (Dc) of the diameter of obliquely displaced particles can be determined. More specifically, the threshold Dc can be determined from the following Equation 1.

$$Dc = 2\eta G\varepsilon \text{ (Equation 1)}$$

where,

Dc: Threshold of obliquely displaced particle diameter
$\eta$: Variable
G: Inter-pillar gap
$\varepsilon$: Pillar offset angle (tan $\theta$)

By solving the above Equation 1, the following approximate Equation 2 is obtained.

$$Dc = 1.4\, G\varepsilon^{0.48} \text{ (Equation 2)}$$

From the empirical rule, the separation is well achieved when $0.06 < \varepsilon < 0.1$, and therefore the following Relationship 3 can be derived from $\varepsilon = \tan\theta = 1/15 = 0.067$

$$G \fallingdotseq 2.62057\, Dc \text{ (Equation 3)}$$

Then, from Equation 3, the inter-pillar gap G is calculated based on the threshold Dc of the diameter of the obliquely displaced particles to produce a flow channel device having a structure in which pillars (obstacle structures) are provided at a specific inter-pillar gap.

In this manner, a flow channel device having a DLD micro-flow channel with an object threshold Dc can be produced.

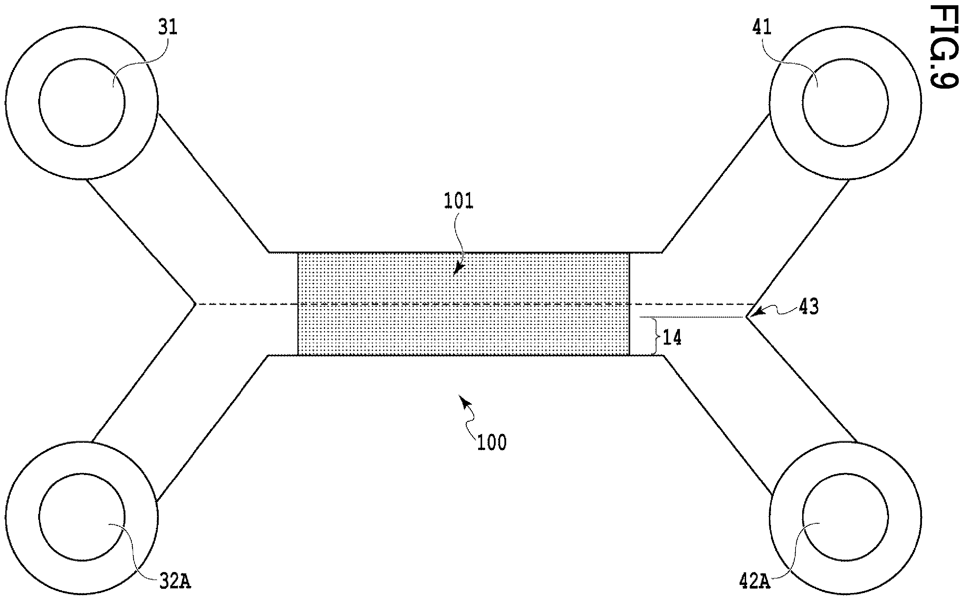

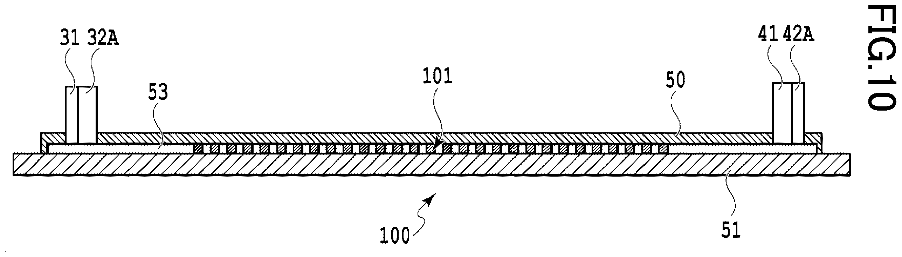

As an example of the flow channel device, Fig. 9 shows a flow channel device 100 provided with a DLD structure 101 based on the DLD as an external force for displacing the object particles may be used. Fig. 10 shows the cross section.

It should be noted that, besides the threshold of the particle diameter, the flow channel length is an important factor for more effectively replacing the liquid medium. Since the displacement distance of the object particles in the lateral direction is proportional to the flow channel length, a flow channel with a larger length is more preferable.

Some embodiments of the present invention have been described so far in detail. The present invention is not limited to these embodiments, but may be modified within a range not departing from the spirit of the present invention, and these modifications should be encompassed by the present invention.

[Examples]

The present example was aimed to separate 30-$\mu$m beads or composite particles of the beads and bioparticles, contained in the suspension and to replace the liquid medium.

(Preparation Example 1) Preparation of a flow channel device

Based on the principles of the deterministic lateral displacement (DLD) method, a flow channel device with a deterministic lateral displacement (DLD) structure was prepared using dimethyl polysiloxane (PDMS). In the device, a particle diameter of 30 $\mu$m was set as the separation threshold Dc.

Specifically, the device had a basic structure in which pillars with a diameter of 15 $\mu$m were arrayed at an interval of 78.6 $\mu$m so as to be offset by one row every 15 pillars. The interval G=78.6 $\mu$m was obtained by substituting Dc=30 $\mu$m into the formula G$\fallingdotseq$2.62057Dc. The offset was based on suitable separation conditions of $\varepsilon=\tan\theta=1/15$.

The height of the flow channel space was set to 50 $\mu$m.

More specifically, the flow channel device was prepared to include the above-described basic structure by preparing a resist mold by using a mask, followed by molding dimethylpolysiloxane (PDMS) to produce a flow channel made of PDMS, in which the pillars were arrayed at equal intervals, thereby forming a DLD-micro flow channel structure 50 having a micro-pillar structure.

Then, the flow channel structure 50 was configured to have port holes at one of both ends thereof to provide a sample liquid (first liquid medium and object particles) inlet 31 and a liquid medium (second liquid medium) inlet 32A. Further, at the other end of the flow channel structure 50, the first outlet 41 and the second outlet 42A were provided. In consideration of preventing sample liquid from directly entering the recovered laminar flow fraction from the first laminar flow segment even when a very slight turbulence occurred in the vicinity of the outlet, a partition wall 43 between the first outlet 41 and the second outlet 42A was positioned such that the laminar flow fraction recovery surface of the second outlet 42A perpendicular to the flow direction of the laminar flow was separated from the cross section of the first laminar flow segment composed of the sample liquid.

Next, the flow channel structure 50 was connected to a glass substrate which served as the planar structure 51, thereby forming a flow channel space 52 in a space between the flow channel structure and the glass substrate.

Then, tubes were respectively attached to the sample liquid inlet 31, the buffer liquid medium inlet 32A, and the first outlet 41 and the second outlet 42A to prepare a flow channel device having a liquid feeding unit.

This flow channel device was used in Examples 1-3 below.

The ratio of the flow channel widths of the first and second laminar flow segments of the flow channel device (x1:y1, Fig. 4) was approximately 1:1.

(Example 1)

A first liquid medium in which 30-$\mu$m sample beads as object particles were suspended [phosphate buffer solution (PBS) containing 5% of bovine serum albumins (BSA)] was prepared as a sample liquid, and a phosphate buffer solution (PBS) was prepared as a second liquid medium to attempt replacement of the liquid medium by displacing the sample beads from the first liquid medium to the second liquid medium by using the flow channel device prepared in Preparation Example 1.

First, before the sample liquid and the second liquid medium were fed, a phosphate buffer solution (PBS) containing 0.05% of polyoxyethylene sorbitan fatty acid ester (Tween) was supplied to the sample liquid inlet 31 and the liquid medium inlet 32A. Then, bubbles were removed from the flow channel, followed by supplying the phosphate buffer solution (PBS) to fill the flow channel device with the phosphate buffer solution (PBS) which was the second liquid medium.

Next, the sample liquid and the second liquid medium were respectively fed from the sample liquid inlet 31 and the liquid medium inlet 32A by a syringe pump at a constant rate of 10 $\mu$L/min, after which formation of a laminar flow constituted by a first laminar flow segment composed of the sample liquid, and a second laminar flow segment composed of the second liquid medium was confirmed.

In microscopic observation of the discharged liquid obtained from the first outlet 41, and the discharged liquid obtained from the second outlet 42A, it was found that the 30-$\mu$m beads were not contained in the discharged liquid obtained from the first outlet 41 but were contained in the discharged liquid obtained from the second outlet 42A (recovered laminar flow fraction).

Further, in quantification of protein of each supernatant of the discharged liquids by the bicinchoninic acid (BCA) assay, 5% of proteins were detected in the supernatant of the discharged liquid obtained from the first outlet 41. However, in the supernatant of the discharged liquid obtained from the second outlet 42A, substantially no protein was detected.

As described above, it was confirmed that the liquid medium was replaced by displacing the 30-$\mu$m beads dispersed in the first liquid medium into the second liquid medium by using the flow channel device of Preparation Example 1.

(Comparative Example 1)

30-μm beads suspended in PBS containing 5% of BSAs were taken into a tube, and were centrifuged at 1000×g (gravitational acceleration), followed by adding PBS to replace the liquid medium. As a result, the amount of the 30-μm beads was reduced, and when a BCA (bicinchoninic acid) was assayed, a small amount of protein was detected from the supernatant.

(Example 2)

30-μm beads labelled with anti-CEA monoclonal antibody was reacted with carcinoembryonic antigens (CEA, Model "HISCL CEA Calibrator", Kinos) suspended in PBS containing 5% of BSAs (first fluid medium) at concentrations of 10 ng/mL to form composite particles.

Similar to Example 1, as for the sample liquid thus obtained, the liquid medium was attempted to be replaced by displacing the composite into a separately prepared PBS (second liquid medium) by using the flow channel device prepared in Preparation Example 1.

First, before feeding the sample liquid and PBS, a PBS buffer solution containing 0.05% of Tween was supplied to the sample liquid inlet 31 and the liquid medium inlet 32A, followed by removing air bubbles from the flow channel to fill the device.

Next, the sample liquid and the PBS were respectively fed from the sample liquid inlet 31 and the liquid medium inlet 32A by a syringe pump at a constant rate of 10 μL/min, after which formation of a laminar flow constituted by a first laminar flow segment composed of the sample liquid, and a second laminar flow segment composed of the second liquid medium was confirmed.

In microscopic observation of the discharged liquid obtained from the first outlet 41 and the discharged liquid obtained from the second outlet 42A, it was found that the 30-μm beads were not contained in the discharged liquid obtained from the first outlet 41, but were contained in the discharged liquid obtained from the second outlet 42A.

Further, in quantification of protein of each supernatant of the discharged liquids by the BCA assay, approximately 5% of proteins were detected in the supernatant of the discharged liquid obtained from the first outlet 41. However, in the supernatant of the discharged liquid obtained from the second outlet 42A, substantially no protein was detected.

As described above, it was confirmed that the first liquid medium was replaced with PBS by displacing the 30-μm beads composite into the second liquid medium using the flow channel device of Preparation Example 1.

Since the size of the antigen used in this example is considerably smaller than the size of the beads, even if the composite particles are formed as in this example, the size of the composite particles does not greatly differ from the size of the beads.

(Comparative Example 2)

For the same purposes described above, 30-μm sample beads labelled with anti-CEA monoclonal antibody were reacted with CEA antigens (type "HISCL CEA Calibrator", Kinos) suspended at a concentration of 10 ng/mL in PBS containing 5% of BSAs to form a composite.

The sample liquid thus obtained was taken into a tube, and centrifuged at 1000×g, followed by adding PBS to replace the liquid medium. As a result, the amount of the 30-μm beads was reduced, and when BCA (bicinchoninic acid) was assayed, a small amount of protein was detected from the supernatant.

(Example 3)

This example was conducted for the purpose of replacing whole blood (first liquid medium) with PBS by separating human breast cancer-derived cells (MCF-7) intruded into the whole blood and displacing the cells to a separately prepared PBS (second liquid medium).

First, human breast cancer-derived cells (MCF-7: DS Pharma Biomedical) that had been stained in advance with nucleated cell staining reagents (Hoechst 33342 solution: Takara Bio) as probes were added to normal whole blood diluted in PBS to obtain a sample.

To this sample, anti EpCAM antibody-labeled beads (CD326(EpCAM), Human, pluriBeads, s-beads:pluriselect) having a particle diameter of 30 μm carrying a monoclonal antibody (anti EpCAM antibody) to human epithelial cell adhesion molecule (EpCAM) as the target capture molecule was added, followed by forming a composite by incubating the sample at room temperature with stirring to obtain a sample liquid.

As for this sample liquid, the liquid medium thereof was attempted to be replaced by displacing the composite into a separately prepared PBS (second liquid medium) by using the flow channel device prepared in Preparation Example 1.

First, the device was filled in advance with PBS containing 1% of BSA, and the sample liquid and the PBS were respectively fed from the sample liquid inlet 31 and the liquid medium inlet 32A by a syringe pump at a constant rate of 10 μL/min, after which formation of a laminar flow constituted by a first laminar flow segment composed of the sample liquid and a second laminar flow segment composed of the PBS was confirmed.

In fluorescence microscopic observation of the discharged liquid obtained from the first outlet 41, and the discharged liquid obtained from the second outlet 42A, it was observed that the discharged liquid obtained from the first outlet 41 contained no 30-μm beads nor the composites of the beads and the cells, but contained blood cells and blood components. Further, in fluorescence microscopic observation of the discharged liquid obtained from the second outlet 42A, it was confirmed that the discharged liquid obtained from the second outlet 42A contained the 30-$\mu$m beads and composites of the beads and cells.

As described above, it was confirmed that the first liquid medium was replaced with PBS by displacing the composites of 30-$\mu$m beads into the PBS by using the flow channel device of Preparation Example 1.

The size of the MCF-7 cells used in this example is less than 30 $\mu$m, and the separation threshold is set to be 30 $\mu$m. In this case, only the cells which are the composite particles of the MCF-7 cells and the 30-$\mu$m beads are laterally displaced. If there are beads-unbound cells, the unbound cells are not laterally displaced and exit from the first outlet 41. Therefore, it is preferable to use composite particles in which nearly all the cells in the sample liquid are bonded to the beads by using the beads excessively to minimize loss and increase the recovery rate.

(Comparative Example 3)

For the same purpose as described above, the sample liquid prepared in Example 3 was taken into a tube, and was centrifuged at 1000×g, followed by adding PBS to replace the liquid medium. Compared with Example 3, the amounts of the 30-$\mu$m beads and the composite with the cells thereof were reduced, and the erythrocytes and the like intruded. Therefore, the object composite particles were not be replaced with the PBS liquid medium with high accuracy.

(Preparation Example 2)

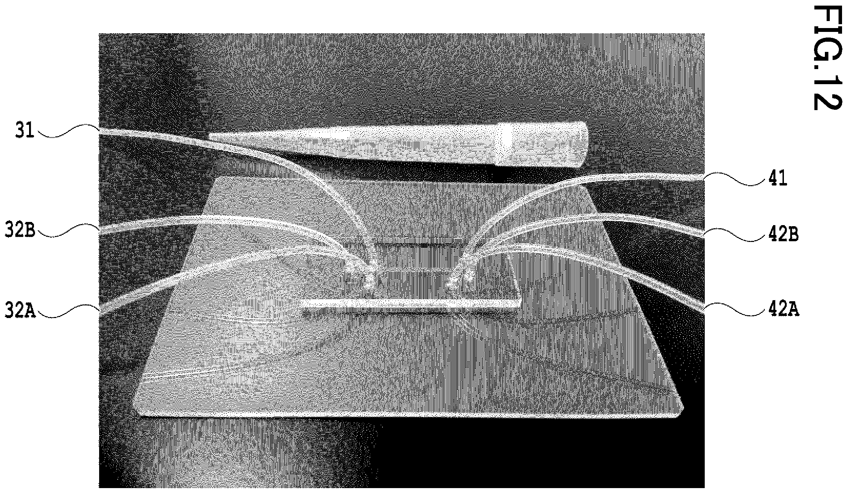

Next, a device shown in Figs. 11 to 14 having three inlets, although having the same micro-pillar structure as that of the flow channel device prepared in Preparation Example 1, was designed, and was prepared according to the same preparation method.

This flow channel device was used in the following Examples 4 to 6.

As also shown in Fig. 14, in this flow channel device, the first laminar flow segment composed of the sample liquid is exclusively discharged from the first outlet 41, and since the third outlet 42B is interposed between the second outlet 42A for recovering the laminar flow fraction containing the object particles and the first outlet 41, the laminar flow fraction recovery surface is clearly greatly separated from the cross section of the first laminar flow segment.

To confirm formation of the laminar flow, first, PBS containing 0.05% of Tween was supplied to the sample liquid inlet 31 and the two liquid medium inlets 32A and 32B in advance, followed by removing air bubbles from the flow channel, and then PBS was supplied to each of the inlets to fill the flow channel devices with PBS.

Next, PBS containing 5% of BSAs was fed from the sample liquid inlet 31 by a syringe pump at a constant speed of 10 $\mu$L/min, PBS was fed from the liquid medium inlet 32A by a syringe pump at a constant rate of 10 $\mu$L/min, and ultrapure water was fed from the liquid medium inlet 32B by a syringe pump at a constant rate of 100 $\mu$L/min, which was 10 times the volume.

Then, a laminar flow as shown in Fig. 14 was formed, the laminar flow being constituted of a first laminar flow segment composed of the sample liquid, third and fourth laminar flow segments composed of ultrapure water respectively located on both sides of the first laminar flow segment, and a second laminar flow segment composed of PBS.

The reason why the liquid feeding speed from the liquid medium inlet 32B is set to 10 times the liquid feeding speed from the liquid medium inlet 32A is that the flow rate of the overall laminar flow is considered to be uniform by setting the liquid feeding amount to an amount proportional to the flow channel volume.

Hereinafter, an experiment was carried out at this liquid feed speed ratio.

(Example 4)

Similar to Example 1, a first liquid medium (PBS containing 5% of BSAs) in which 30-$\mu$m sample beads which were object particles were suspended was prepared as a sample liquid, and PBS was prepared as a second liquid medium.

Then, the liquid medium was attempted to be replaced by displacing the sample beads from the first liquid medium to the second liquid medium by using the flow channel device prepared in Preparation Example 2.

First, before feeding the samples and the PBS, a PBS buffer containing 0.05% of Tween was supplied to the sample liquid inlet 31 and the two liquid medium inlets 32A and 32B, followed by removing air bubbles from the flow channel, and then PBS was supplied to each of the inlets to fill the flow channel devices with PBS.

Next, the sample liquid and the PBS were respectively fed from the sample liquid inlet 31 and the liquid medium inlet 32A by a syringe pump at a constant rate of 10 $\mu$L/min, and the PBS was fed from the liquid medium inlet 32B by a syringe pump at a constant rate of 100 $\mu$L/min, after which formation of a laminar flow was confirmed.

Thereafter, in microscopic observation of the discharged liquid obtained from the first outlet 41, and the discharged liquid obtained from the second outlet 42A, it was found that the 30-$\mu$m beads were not contained in the discharged liquid obtained from the first outlet 41, but instead were contained in the discharged liquid obtained from the second outlet 42A.

In addition, the 30-$\mu$m beads were not contained in the discharged liquid obtained from the third outlet 42B.

Further, in quantification of protein of each supernatant of the discharged liquids by the BCA assay, approximately 1% of protein was detected in the supernatant of discharged liquid obtained from the first outlet 41. However, in the supernatant of discharged liquid obtained from the second outlet 42A, substantially no protein was detected.

Further, compared with the result in Example 1, the supernatant contained fewer proteins.

As described above, it was confirmed that the liquid medium was more accurately replaced by displacing the 30-μm sample beads dispersed in the first liquid medium into the PBS by using the flow channel device of Preparation Example 2.

(Example 5)

Similar to Example 2, 30-μm sample beads labelled with anti-CEA monoclonal antibody were reacted with carcinoembryonic antigens (CEA, Model "HISCL CEA Calibrator", Kinos) suspended in PBS containing 5% of BSAs (first fluid medium) at concentrations of 10 ng/mL to form composite particles.

Similar to Example 4, as for the sample liquid thus obtained, the liquid medium thereof was attempted to be replaced by displacing the composite particles into a separately prepared PBS (second liquid medium) by using the flow channel device prepared in Preparation Example 2.

First, before feeding the sample liquid and PBS, a PBS containing 0.05% of Tween was supplied to the sample liquid inlet 31 and the two liquid medium inlets 32A and 32B in advance, followed by removing air bubbles from the flow channel to fill the device.

Next, the sample liquid and the PBS were respectively fed from the sample liquid inlet 31 and the liquid medium inlet 32A by a syringe pump at a constant rate of 10 μL/min, and the PBS was fed from the liquid medium inlet 32B by a syringe pump at a constant rate of 100 μL/min, after which formation of a laminar flow was confirmed.

In visual observation of the discharged liquid obtained from the first outlet 41, and the discharged liquid obtained from the second outlet 42A, it was visually confirmed that the 30-μm beads were not contained in the discharged liquid obtained from the first outlet 41, but were contained in the discharged liquid obtained from the second outlet 42A. In addition, the 30-μm beads were not contained in the discharged liquid obtained from the third outlet 42B.

Further, in quantification of protein of each supernatant of the discharged liquids by the BCA assay, approximately 1% of protein was detected in the supernatant of discharged liquid obtained from the first outlet 41. However, in the supernatant of discharged liquid obtained from the second outlet 42A, substantially no protein was detected. Further, compared with the result in Example 2, the supernatant contained fewer proteins.

As described above, it was confirmed that the first liquid medium was more accurately replaced with PBS by displacing the 30-μm beads into the PBS by using the flow channel device of Preparation Example 2.

(Example 6)

Similar to Example 3, this example was conducted for the purpose of replacing whole blood (first liquid medium) with PBS by separating human breast cancer-derived cells (MCF-7) intruded into the whole blood, and displacing them to a separately prepared PBS (second liquid medium).

First, human breast cancer-derived cells (MCF-7: DS Pharma Biomedical) that have been previously stained with nucleated cell staining reagents (Hoechst 33342 solution: Takara Bio) as probes were added to normal whole blood diluted in PBS to obtain a sample.

To this sample, anti EpCAM antibody-labeled beads (CD326(EpCAM), Human, pluriBeads, s-beads:pluriselect) having a particle diameter of 30 μm carrying a monoclonal antibody (anti EpCAM antibody) to human epithelial cell adhesion molecule (EpCAM) as the target capture molecule was added, followed by forming composite particles by incubating the sample at room temperature with stirring to obtain the sample liquid.

As for this sample liquid, the liquid medium thereof was attempted to be replaced by displacing the composite particles into a separately prepared PBS (second liquid medium) by using the flow channel device prepared in Preparation Example 2.

First, the flow channel device was filled in advance with PBS containing 1% of BSA in advance, and then the sample liquid and the PBS were respectively fed from the sample liquid inlet 31 and the liquid medium inlet 32A by a syringe pump at a constant rate of 10 μL/min, and the PBS containing 1% of BSA was fed from the liquid medium inlet 32B by a syringe pump at a constant rate of 100 μL/min

In fluorescence microscopic observation of the discharged liquid obtained from the first outlet 41, and the discharged liquid obtained from the second outlet 42A, it was observed that the discharged liquid obtained from the first outlet 41 contained no 30-μm beads, but contained blood cells and blood components.

Further, in fluorescence microscopic observation of the discharged liquid obtained from the second outlet 42A, it was confirmed that the discharged liquid obtained from the second outlet 42A contained the 30-μm beads and composite particles of the beads and cells. Compared with the results obtained from Example 3, it was confirmed that the intrusion of blood components (such as leukocytes or erythrocytes) was reduced with higher accuracy.

Further, the discharged liquid obtained from the third outlet 42B contained neither the 30-μm beads nor composite particles of the beads and cells.

As described above, it was confirmed that the first liquid medium was more accurately replaced with PBS by displacing the beads composite particles having a particle diameter of 30 μm into the PBS using the flow channel device of Preparation Example 2, the beads composite particles being intruded into the whole blood.

**(Example 7)**

Further, similar to Example 6, human breast cancer-derived cells (MCF-7) intruded into whole blood were separated, and then displaced to a separately prepared DMEM medium containing 10% of FBSs (second liquid medium), to replace whole blood (first liquid medium) with DMEM medium containing 10% of FBSs (second liquid medium).

That is, the sample liquid prepared in Example 6 was attempted to be replaced by displacing the composite particles to a separately prepared DMEM medium containing 10% of FBSs (second liquid medium) by using the flow channel devices prepared in Preparation Example 2.

First, the flow channel devices were filled with PBS containing 1% BSA in advance, and then the sample liquid was fed from the sample liquid inlet 31, and the DMEM medium containing 10% of FBSs was fed from the liquid medium inlet 32A by a syringe pump at a constant speed of 10 $\mu$L/min, and the PBS containing 1% of BSA was fed from the liquid medium inlet 32B by a syringe pump at a constant rate of 100 $\mu$L/min, after which formation of a laminar flow was confirmed.

In microscopic observation of the discharged liquid obtained from the first outlet 41, and the discharged liquid obtained from the second outlet 42A, it was observed that the discharged liquid obtained from the first outlet 41 contained no 30-$\mu$m beads, but contained blood cells and blood components.

Further, in microscopic observation of the discharged liquid obtained from the second outlet 42A, it was confirmed that the discharged liquid obtained from the second outlet 42A contained the 30-$\mu$m beads and composites of the beads and cells.

Further, the discharged liquid obtained from the third outlet 42B contained neither 30-$\mu$m beads nor composite particles of the beads and cells.

As described above, the beads replaced in the DMEM medium containing 10% of FBSs and the composite particles with the cells thereof can be subjected to the culture without further replacement of the liquid medium or dilution of the liquid medium.

[Industrial Applicability]

The liquid medium replacement method and device of the present invention can be used for separation and purification of nucleic acids, proteins, cells and the like in research applications, diagnostic applications, manufacture of pharmaceuticals and the like.

[Reference Signs List]

1 ... Object particles
11 ... First laminar flow segment (first liquid medium containing dispersed object particles)
12 ... Second laminar flow segment (second liquid medium receiving object particles)
F ... External force
13 ... Third laminar flow segment (third liquid medium)
14 ... Laminar flow fraction recovery surface
15 ... Cross section (perpendicular to the laminar flow) of the first laminar flow segment
30 ... Example of the basic structure of a flow channel device
31 ... Sample liquid inlet (first liquid medium containing dispersed object particles)
32A ... Liquid medium inlet (second liquid medium receiving object particles)
41 ... First outlet
42A... Second outlet (object particles-containing laminar flow fraction recovery outlet)
40 ... Example of another structure of a flow channel device
43 ... Outlet side partition wall
44 ... Inlet side partition wall
32B ... Liquid medium inlet (third liquid medium)
42B ... Third outlet
50 ... Flow channel structure
51 ... Planar structure
53 ... Flow channel space
81 ... Sample liquid feeding unit
82A... Liquid medium feeding unit (second liquid medium feeding unit)
91 ... First recovering unit
92A ... Second recovering unit (object particles-containing laminar flow fraction recovering unit)
100 ... Flow channel device for use in examples
101 ... Deterministic lateral displacement (DLD) structure
x1 ... Width of the first laminar flow segment
y1 ... Width of the second laminar flow segment
x2 ... Discharge width by the first outlet
y2 ... Discharge width by the second outlet

**Claims**

1. A method of replacing a liquid medium by displacing object particles from a first liquid medium to a second liquid medium, comprising:

   (i) a step of forming a laminar flow of a plurality of laminar flow segments by feeding at least two liquids, of a sample liquid and a second liquid medium, in the same direction,
   the sample liquid being a first liquid medium that contains dispersed object particles,
   the laminar flow including at least a first laminar flow segment composed of the sample liquid,

and a second laminar flow segment composed of the second liquid medium;
(ii) a step of displacing the object particles from the first laminar flow segment to the second laminar flow segment by applying an external force to the laminar flow, the object particles having at least one physical characteristic of being allowed to displace towards the second laminar flow segment from the first laminar flow segment by an external force; and
(iii) a step of recovering a laminar flow fraction containing the object particles from the second laminar flow segment, the laminar flow fraction being recovered from a laminar flow fraction recovery surface perpendicular to a flow direction of the laminar flow, and the laminar flow fraction recovery surface being separated from a cross section of the first laminar flow segment.

2. The method according to claim 1, wherein the first laminar flow segment is adjacent to, and in contact with the second laminar flow segment.

3. The method according to claim 1, wherein a third laminar flow segment composed of at least a third liquid medium is interposed between the first laminar flow segment and the second laminar flow segment.

4. The method according to any one of claims 1 to 3, wherein the external force is at least one of external forces due to electric field, magnetic field, hydrodynamic filtration, and deterministic lateral displacement (DLD).

5. The method according to any one of claims 1 to 3, wherein the external force includes at least an external force due to deterministic lateral displacement (DLD), and physical characteristics of the object particles reside in particle diameter.

6. The method according to any one of claims 1 to 5, wherein the object particles include at least one of bioparticles, beads, and composite particles of beads and bioparticles.

7. The method according to claim 6, wherein the object particles are composite particles of bioparticles and beads, and the second liquid medium dissociates the composite particles to liberate the bioparticles.

8. The method according to claim 6 or 7, wherein the bioparticles are one of biomolecules, extracellular vesicles, and cells.

9. The method according to claim 8, wherein the bioparticles are either biomolecules or cells.

10. The method according to claim 9, wherein the bioparticles are cells.

11. A flow channel device that displaces object particles from a first liquid medium to a second liquid medium, comprising:

   (i) a plurality of inlets including at least a first inlet for introducing a sample liquid, and a second inlet for introducing a second liquid medium on an upstream side of the flow channel device;
   (ii) a flow channel that forms a laminar flow;
   (iii) two or more outlets including at least a first outlet and a second outlet on a downstream side of the flow channel device; and
   (iv) a mechanism that applies an external force, wherein:

      the sample liquid is a first liquid medium containing dispersed object particles;
      the flow channel forms a laminar flow including at least a first laminar flow segment composed of the sample liquid, and a second laminar flow segment composed of a second liquid medium;
      the second outlet is an object particle recovery outlet that recovers a laminar flow fraction from a laminar flow fraction recovery surface perpendicular to a flow direction of the laminar flow, the laminar flow fraction including the object particles that has been displaced to the second laminar flow segment by the external force;
      the first outlet is on the first laminar flow segment side as viewed from the second outlet; and
      a partition wall of the second outlet on the first laminar flow segment side is provided such that the laminar flow fraction recovery surface is separated from a cross section of the first laminar flow segment.

12. The flow channel device according to claim 11, wherein the first inlet is adjacent to the second inlet so that the first laminar flow segment and the second laminar flow segment are formed being adjacent to and in contact with each other.

13. The flow channel device according to claim 11, wherein:

      the flow channel device further comprises a third inlet for introducing a third liquid medium, provided on an upstream side of the flow channel device,
      the flow channel further forms a laminar flow including a third laminar flow segment composed of a third liquid medium, and
      the third inlet is provided between the first inlet and the second inlet so that the third laminar flow segment is interposed between the first laminar flow segment and the second laminar flow segment.

14. The flow channel device according to any one of claims 11 to 13, wherein the mechanism that applies an external force has a predetermined pattern structure in which a plurality of columnar structures for utilizing deterministic lateral displacement (DLD) are arrayed.

15. A flow channel device system comprising a plurality of the flow channel devices according to claim 11, wherein

the plurality of the flow channel devices are connected in series,

the serial connection is formed by connecting the second outlet of a first flow channel device to the first inlet of a second flow channel device to be mated, and

the plurality of the flow channel devices have respective mechanisms for applying external force, or respective strengths of external force, which are different between devices.

# FIG.1

FIG.2

EP 3 473 317 A1

FIG.3

# FIG.4

FIG.5

EP 3 473 317 A1

# FIG.6

FIG.7

# FIG.8

FLOW DIRECTION

FIG.9

# FIG.10

FIG.11

# FIG.12

# FIG.13

# FIG.14

STRUCTURE NEAR OUTLET

INTERMEDIATE STRUCTURE OF FLOW CHANNEL DEVICE

STRUCTURE NEAR INLET

## INTERNATIONAL SEARCH REPORT

| International application No. |
| --- |
| PCT/JP2017/022554 |

### A. CLASSIFICATION OF SUBJECT MATTER
*B01D12/00*(2006.01)i, *B01J19/00*(2006.01)i, *B03C1/00*(2006.01)i, *C12M1/00* (2006.01)i, *G01N1/28*(2006.01)n, *G01N37/00*(2006.01)n

According to International Patent Classification (IPC) or to both national classification and IPC

### B. FIELDS SEARCHED

Minimum documentation searched (classification system followed by classification symbols)
B01D11/00-11/04, B01D12/00, B01J19/00-19/32, B01D43/00, B03C1/00-1/32, B81C1/00-7/04, C12M1/00-3/10, C12Q1/00-3/00, G01N1/00-1/34, G01N33/48-33/98, G01N37/00,

Documentation searched other than minimum documentation to the extent that such documents are included in the fields searched
 Jitsuyo Shinan Koho  1922–1996 Jitsuyo Shinan Toroku Koho 1996–2017
 Kokai Jitsuyo Shinan Koho 1971–2017 Toroku Jitsuyo Shinan Koho 1994–2017

Electronic data base consulted during the international search (name of data base and, where practicable, search terms used)
 JSTPlus/JST7580/JSTChina(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2009-511001 A (Artemis Health, Inc.),<br>19 March 2009 (19.03.2009),<br>claims 73 to 147; paragraphs [0083] to [0155],<br>[0176] to [0184]; fig. 1A to 101<br>& WO 2007/035498 A2<br>page 31, line 10 to page 52, line 26; page 58,<br>line 24 to page 60, line 7; page 98, line 8 to<br>page 106, line 13; fig. 1 to 101 | 1-6,8-15<br>7 |
| Y | JP 2011-522219 A (Valtion Teknillinen Tutkimuskeskus),<br>28 July 2011 (28.07.2011),<br>claims; paragraphs [0057], [0100]; fig. 1 to 7<br>& WO 2009/125067 A1<br>page 20, lines 2 to 20; page 31, line 30 to<br>page 32, line 24; pages 39 to 44; fig. 1 to 7 | 7 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br> 14 July 2017 (14.07.17) | Date of mailing of the international search report<br> 01 August 2017 (01.08.17) |
| --- | --- |
| Name and mailing address of the ISA/<br> Japan Patent Office<br> 3-4-3,Kasumigaseki,Chiyoda-ku,<br> Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2017/022554 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | JP 2012-529983 A (Commissariat a l'Energie Atomique et aux Energies Alternatives), 29 November 2012 (29.11.2012), claims; paragraphs [0001] to [0102]; fig. 1 to 30 & US 2012/0125842 A1 pages 1 to 11; fig. 1 to 30 & WO 2010/146261 A2 | 1-6,8-14 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D.W.INGLIS et al.** Critical particle size for fractionation by deterministic lateral displacement. *Lab on a Chip,* 2006, vol. 6, 655-658